# EUROPEAN PATENT SPECIFICATION

(11) **EP 3 419 607 B1**
(45) Date of publication and mention of the grant of the patent: **20.11.2019**
(21) Application number: 17716641.0
(22) Date of filing: 29.03.2017
(51) Int. Cl.: A61K 9/48, A61K 31/137, A61P 25/00

(54) **STABLE FORMULATIONS OF FINGOLIMOD**
STABILE FORMULIERUNGEN VON FINGOLIMOD
FORMULATIONS STABLES DE FINGOLIMOD

(43) Date of publication of application: 02.01.2019
(73) Proprietor: Deva Holding Anonim Sirketi, 34303 Istanbul (TR)
(72) Inventor: HAAS, Philipp Daniel, 34303 Istanbul (TR); STECKEL, Hartwig Andreas, 34303 Istanbul (TR); AVCI, Recep, 34303 Istanbul (TR); KANDEMIR, Levent, 34303 Istanbul (TR)
(86) International application number: PCT/IB2017/051788
(87) International publication number: WO 2018/178744

(56) References cited:
- WO-A1-2016/042493
- IN-A- 3427M UM2 013
- IN-A- 3428M UM2 013

## Description

### Field of the Invention

The present invention relates to stabilized pharmaceutical composition comprising fingolimod, or pharmaceutically acceptable salts, esters, hydrates and solvates thereof, process of preparation and method of using the same.

More particularly, the present invention provides a stable solid pharmaceutical composition suitable for oral administration, comprising fingolimod or pharmaceutically acceptable salts, esters, hydrates and solvates thereof and calcium carboxymethylcellulose.

### Background Art

Multiple sclerosis is the most common inflammatory demyelinating disease of the central nervous system (CNS) which affects more than 1.2 million people worldwide.

Multiple sclerosis (MS) is a disease in which the nerves of the central nervous system (brain and spinal cord) degenerate. Myelin provides a covering or insulation for nerves, improves the conduction of impulses along the nerves and also is important for maintaining the health of the nerves. In multiple sclerosis, inflammation causes the myelin to disappear. Consequently, the electrical impulses that travel along the nerves decelerate. In addition, the nerves themselves are damaged. As more and more nerves are affected, patient suffers from a range of symptoms which affect their health related quality of life such as pain, muscle spasticity and spasm, bladder problems and sleep disturbance.

Spasticity is a common symptom of multiple sclerosis and as the disease evolves, it occurs in more than 60% of people with MS. Available treatments are deemed to be partial relief for spasticity and have unpleasant side effects.

Although there is no known cure for multiple sclerosis, there are few effective treatments for the symptoms of multiple sclerosis. Marketed medications are aimed to reduce the frequency of attacks or attempt to return functions after an attack or prevent new attacks. Nevertheless, it is reported that only a minority of people can benefit from current drugs and associated adverse side effects significantly limit use of these drugs.

Despite the introduction of some different medications for the treatment of multiple sclerosis over years, there is a very clear needs for alternative new treatments for MS.

Fingolimod's oral multiple sclerosis therapy is a significant step for patients and addresses the unmet need for additional therapies.

Fingolimod which is also referred to as "FTY720" is a synthetic imitation of myriocin; a metabolic product of the fungus Isaria sinclairii.

Fingolimod-phosphate is a sphingosine 1-phosphate receptor modulator and it is metabolized by sphingosine kinase to the active metabolite, fingolimod-phosphate. Fingolimod binds with high affinity to sphingosine 1-phosphate receptors 1, 3, 4, and 5.The IUPAC name of fingolimod is 2-amino-2-(2-[4-octylphenyl] ethyl)-1,3-propane diol and its chemical structure is shown below:

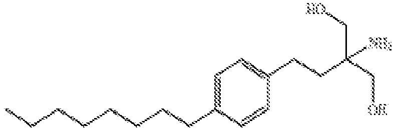

Fingolimod which is a first line or a second line treatment; is indicated for the treatment of patients with relapsing forms of MS to reduce the frequency of clinical exacerbations and severity of symptoms in MS and delay the accumulation of physical disability. Since its market introduction, fingolimod is assessed to be promising and hopeful for several patients with regard to other medications.

Fingolimod is the first-in-class orally available drug for the treatment of relapsing multiple sclerosis. Fingolimod is currently marketed as an immediate release capsule for the treatment of multiple sclerosis under the trade name Gilenya®. It is formulated as 0.5 mg hard capsules to be taken once daily and offers patients an alternative to the currently available injectable therapies.

Gilenya™ capsule comprises mannitol as filler, prepared by direct blending method and the capsule additionally comprises small amount of magnesium stearate as a lubricant.

It is disclosed in literature that fingolimod reacts with several excipients during compatibility studies and thus mannitol was used as filler in Gilenya™ formulation due to the optimum degradation profile.

Various formulations corresponding to stable fingolimod's finished dosage forms have been disclosed in the literature.

EP 0627406 B1 (YOSHITOMI PHARMACEUTICAL INDUSTRIES LTD.) 28.10.1998 firstly discloses 2-amino-1,3-propane-diol compounds.

US 6,004,565 (YOSHITOMI PHARMACEUTICAL) 21.12.1999 discloses method for accelerating the lymphocyte homing activity of the immune system in a mammal by administering fingolimod hydrochloride.

EP1613288 B1 (NOVARTIS AG) 19.11.2008 discloses solid pharmaceutical composition for oral administration comprising fingolimod and a sugar alcohol.

WO2012/135561(NOVARTIS AG) 04.10.2012 discloses a solid oral pharmaceutical composition comprising fingolimod, a filler and a cyclodextrin as a stabilizer.

WO2013/019872 (TEVA PHARMA) 07.02.2013 discloses pharmaceutical composition comprising an intimate admixture of fingolimod and a solid surfactant.

EP2560621 A1 (RATIOPHARM GMBH) 27.02.2013 discloses an intermediate containing fingolimod and matrix material, wherein the fingolimod is present in the matrix material in the form of a solid solution.

WO 2013/091704(SYNTHON BV) 27.06.2013 discloses a pharmaceutical composition comprising fingolimod, calcium lactate pentahydrate and optionally a lubricant. US 8,673,918 (NOVARTIS AG) 18.03.2014 discloses dosage forms containing fingolimod and one or more excipients selected one or more from fillers(e.g. lactose monohydrate, lactose anhydrous, maize starch, sucrose, and microcrystalline cellulose, citric acid and sodium hydrogen carbonate), binders, disintegrants, lubricants, flow regulators, matrix formers.

Studies of drug-excipients compatibility represent an important phase in the preformulation stage of the development of all dosage forms. The potential physical and chemical interactions between drugs and excipients can affect the chemical, physical, therapeutical properties and stability of the dosage form.

In the present case, since the dose of the approved product is very low, and filler makes most of the part of the composition, it is essential to select excipient which is highly compatible with active pharmaceutical ingredient.

### Summary of invention

It is an object to provide a pharmaceutical composition suitable for oral administration of fingolimod, or pharmaceutically acceptable salts, esters, hydrates and solvates thereof, which composition exhibits improved stability upon long-term storage and has advantageous handling properties in making orally administrable final dosage forms such as capsules or tablets.

### Technical problem

Fingolimod is an effective low dose immunosuppressant agent; the recommended dosing regimen of fingolimod is 0.5 mg once a day in oral administration. The pharmaceutical product development of such low-dose drugs generally poses major problems. Non homogeneity of the blend, powder segregation and poor flowability of the powder are main challenges to overcome when formulating and manufacturing low dose drugs such as fingolimod.

The small amount of active ingredient that is typically used for the manufacture of these low dose units must first be evenly distributed in a powder blend. Since the quantity of active ingredient is low in formulation; unacceptable blend and content uniformity are usually encountered in tablet or capsule dosage forms.

The inventors have also discovered that fingolimod particles have a strong tendency to stick to the processing equipments and to each other and thus cause stability and blend uniformity problems.

Furthermore, fingolimod can react with certain excipients to produce degradation products in the final formulation. For this reason, only a limited number of pharmaceutical excipients, particularly fillers, can be suitable.

The inventors have further found that the stability and blend/content uniformity of pharmaceutical compositions containing fingolimod are heavily dependent on the choice of excipients used in the formulation, and the manufacturing process by which the formulation is prepared.

According to disclosures in the prior art, fingolimod being an amino substituted product has content uniformity issue sand stability issues as a result due to Maillard reaction of amino groups with sugar alcohol. The Maillard reaction is a chemical reaction between an amino acid and a reducing sugar (Sugars that contain aldehyde groups that are oxidized to carboxylic acids are classified as reducing sugars. Reducing sugars include glucose, glyceraldehyde, lactose, arabinose and maltose), usually requiring the addition of heat.

Thus, due to low-dose, stability, blend uniformity, content uniformity, manufacturing processing issues in the prior art, there is still a need to develop stabilized formulations comprising fingolimod which at the same time address the issues present in the prior art as well as result in a formulation which are more robust, economical, have good storage stability, are less time consuming and have comparable dissolution and bioavailability with respect to the marketed product Gilenya® (fingolimod hydrochloride capsules).

In particular, there is a need for a stable pharmaceutical composition of fingolimod, which are stable throughout the shelf life. The present invention relates to a stable pharmaceutical composition of fingolimod and its preparation.

### Solution to Problem

According to disclosures in the prior art, various SIP receptor modulators comprising an aminopropane-1,3-diol group (such as fingolimod)are not easy to formulate in solid oral formulation due to Maillard reaction; only a limited number of excipients are potentially feasible with such amino-diols. In the present invention,

After various approaches used to address the blend and content uniformity problems in f low-dose solid dosage forms, the inventors havealso managed to formulate fingolimod which comprises an amino-propane-1,3-diols by preventing Maillard reaction.

According to the present invention, the calcium carboxymethylcellulose is adopted as the filler agent and the stabilizing agent so as to increase the stability of the dosage form, effectively delay fingolimod's degradation and ensure drug quality. Thus, it is possible to resolve ongoing problems and make improvement over the commercially available drug by using only one excipient.

Presented formulations surpass previous problems arising when formulating low dose of fingolimod due to homogeneously distribution by geometric dilution with calcium carboxymethylcellulose.

The inventors have also discovered that calcium carboxymethylcellulose formed stable compositions with fingolimod or pharmaceutically acceptable salts, esters, hydrates and solvates thereof.

The stability of such compositions in long-term storage tests is at least comparable with that of similar compositions with mannitol which is considered in the prior art as the most suitable filler for formulating fingolimod into oral pharmaceutical compositions, and it is superior to many other earlier suggested fillers.

The compositions of the present invention exhibit good handling properties, e.g. flowability, content uniformity etc. for making powders or granulates for both direct oral administration or for tabletting.

### Description of embodiments

Fingolimod is indicated for the treatment of patients with relapsing forms of multiple sclerosis (MS) to reduce the frequency of clinical exacerbations and to delay the accumulation of physical disability.

In the present invention, the present inventors have aimed to provide stable pharmaceutical compositions containing fingolimod, or pharmaceutically acceptable salts, esters, hydrates and solvates thereof, for oral administration having a good dissolution profile, an improved physical stability and good blend and content uniformity results.

In the first aspect of the invention, the present inventors have aimed to improve stability, blend uniformity and performed different manufacturing process.

During studies, it is noticed that the fingolimod can react with certain excipients to produce degradation products in the finished dosage form. Thus, the selection of excipients is vital in the design of a quality drug product. Claimed excipients and its concentration in a formulation are selected based not only on their functionality but also on the compatibility between the active ingredient and excipient.

In the present invention, it is necessary to select suitable filler in order to overcome stability and blend uniformity problems. In order to obtain less degradation products, several types of filler have been investigated and only calcium carboxymethylcellulose was found to be the most effectual filler in controlling the stability of fingolimod.

The inventors of the present invention have conducted stability analysis , and the following finished dosages have been compared accordingly; the commercially available product comprising fingolimod and sugar alcohol (Gilenya™), the pharmaceutical composition comprising fingolimod and microcrystalline cellulose and pharmaceutical composition comprising fingolimod and calcium carboxymethylcellulose.

On the other hand, the present inventors have chosen microcrystalline cellulose as comparative filler because microcrystalline cellulose is found to be one of the most suitable filler for fingolimod composition according to intensive studies.

The stability results of the pharmaceutical compositions studied for three months of accelerated stability at 40°C± 2°C, at 75% ± 5% relative humidity are given in Table 1.

**Table 1**

| | Initial | | | 3^{rd} month results of accelerated stability conditions (40°C± 2°C, 75% ± 5% Rh) | | |
|---|---|---|---|---|---|---|
| | Gilenya® 0.5 mg Hard Capsule | Fingolimod 0.5 mg Capsule (Example 3) | Fingolimod 0.5 mg Capsule (Example 5) | Gilenya® 0.5 mg Hard Capsule | Fingolimod 0.5 mg Capsule (Example 3) | Fingolimod 0.5 mg Capsule (Example 5) |
| | | (calcium carboxymet hycellulose used as filler) | (microcrystalline cellulose used as filler) | | (calcium carboxymet hycellulose use d as filler) | (microcrystalline cellulose used as filler) |
| **Assay** Fingolimod | 0.498 mg | 0.499 mg | 0.490 mg | 0.490 mg | 0.496 mg | 0.480 mg |

| **Degradation Products** | | | | | | |
|---|---|---|---|---|---|---|
| FIM 6 (Degradation product H) | Not detected | Not detected | Not detected | Not detected | Not detected | Not detected |
| Any unspecified degradation product | 0.42% | 0.12% | 0.22% | 0.67% | 0.19% | 0.44% |
| Total degradation products | 0.42% | 0.42% | 0.61% | 0.95% | 0.66% | 1.49% |

The obtained results, as shown in Table 1, clearly indicate that the pharmaceutical composition containing fingolimod and calcium carboxymethylcellulose has given better stability results than commercially available product comprising sugar alcohol (Gilenya™) and pharmaceutical composition comprising microcrystalline cellulose.

Advantageously, stable pharmaceutical compositions of fingolimod having good blend uniformity can be prepared by using calcium carboxymethylcellulose according to the present invention.

Preferably, pharmaceutical compositions prepared according to the invention have a stability such that after storage at 40 °C±2°C at 75%± 5% relative humidity for 3 months, the total amount of impurities and degradation products does not increase by more than 3%, preferably does not increase by more than 2.5%, and more preferably does not increase by more than 2% of the initial total amount of impurities and degradation products.

The inventors of the present invention have conducted further long-term stability analysis of commercially available product comprising fingolimod and sugar alcohol (Gilenya™) and pharmaceutical composition comprising fingolimod and calcium carboxymethylcellulose.

As a result of formulation discovered by present inventors, the long term stability values are give better results as well as three months stability values The stability results of the pharmaceutical compositions studied for six months at 40°C± 2°C, at 75% ± 5% relative humidity are given in Table 2.

**Table 2**

| | 6^{th} month results of accelerated stability conditions (40°C± 2°C, 75% ± 5% Rh) | |
|---|---|---|
| | Gilenya® 0.5 mg Hard Capsule | Claimed Fingolimod 0.5 mg Capsule |
| FIM 6 (Degradation product H) | Not detected | Not detected |
| Any unspecified degradation product | 0.67% | 0.19% |
| Total degradation products | 1.18% | 0.78% |

The obtained results, shown in Table 2, clearly indicate that the total amount of impurities and degradation products of the present composition (fingolimod and calcium carboxymethylcellulose) contains less the total impurity and degradation products than the composition containing sugar alcohol.

Inventor's excessive studies have revealed that fingolimod, or pharmaceutically acceptable salts, esters, hydrate and solvates thereof, is formulated in very stable and homogenous composition with calcium carboxymethylcellulose.

It is known that the quality of the solid dosage form is dependent on the adequacy of the blend. Producing a uniform mixture of the drug and its excipients is paramount in being able to deliver the proper dose of the drug to the patient. Once an adequate blend is obtained, it is also critical to ensure that it does not segregate in the post blending handling steps. Millions of dosage units may be created from a single batch, and each and every dose must be of acceptable composition, to ensure the safety and efficiency of the product. Therefore, the homogeneity of pharmaceutical blends and dosage units is highly scrutinized by regulatory bodies throughout the world.

The homogeneity of the present pharmaceutical composition is obtained when fingolimod is diluted with calcium carboxymethylcellulose by using geometric dilution process.

Geometric dilution is a technique to aid in the distribution of smaller quantities of active ingredients into the larger bulk of the blend. It involves blending a quantity of the active ingredient (X) with an equal part of diluent(X).Once blended; an additional quantity of diluents equal to the total quantity already blended in the first step (2X) is added to the previous blend and blended. This process is repeated by adding 4X, 8X, 16X, etc. Quantities of diluents until the active ingredient are sufficiently diluted to be incorporated into the remainder of the batch. Geometric dilution can be done in a single blender, or may require the use of a larger blender for the combination of larger quantities in the procedure. In its simplest application, geometric dilution is used by pharmacists during the extemporaneous compounding of a prescription, typically using mortar and pestle to uniformly incorporate the drug into the remaining formulation components. On a large scale, drug manufacturers use geometric dilution during the manufacture of both pilot and large-scale batches of solid dosage forms. The same principle applies in each case, with the only difference being the type of equipment used to accommodate the quantities of materials being processed.

The powder blending process has been historically identified as a challenging operation in oral solid dosage form's manufacturing. Insufficient blending results in poor active ingredient mixing with excipients. Excessive blending could adversely impact the distribution of active ingredient's content in the final product.

In other words, the powder blending is a fundamental process of mixing for solid dosage forms to ensure content uniformity of final drug products. The powder blend uniformity is a process control (i.e., in-process material test) that critically impacts the uniformity of dosage form.

Blending includes producing an adequate blend, maintaining that blend through additional handling steps, and verifying that both the blend and the finished product are sufficiently homogeneous. Therefore, a complete approach should be used to assess the uniformity of blends, and the subsequent dosage forms produced from them.

In one aspect, the present invention relates to a process for producing a pharmaceutical composition, comprising:
(1)Dispensing raw materials separately.
(2)Sifting fingolimod HCl and filler through a suitable sieve and load into the container blender.
(3)Blending the sifted mixture for 15 minutes at 20 rpm.
(4)Sifting the lubricant through a suitable sieve and add into the container blender and blending it for 5 minutes at 20 rpm.
(5)Filling the final mixture in a suitable size hard gelatin capsules with 50.0 mg ± 5.8% (47.1 mg - 52.9 mg) average fill weight and certain capsule filling parameters.

In second aspect, the present invention relates to a process for producing a pharmaceutical composition, comprising:
(1)Dispensing raw materials separately.
(2)Blending fingolimod HCl and one third (1/3) of filler in an appropriate container for 5 minutes.
(3)Sifting the obtained mixture with remaining filler (2/3) through a suitable sieve and loading into the container blender.
(4)Blending the sifted mixture for 15 minutes at 20 rpm.
(5) Sifting the lubricant through a suitable sieve and add into the container blender and blending it for 5 minutes at 20 rpm.
(6) Filling the final mixture in suitable size hard gelatin capsules with 50.0 mg ± 5.8 % (47.1 mg - 52.9 mg) averages fill weight and certain capsule filling parameters.

Present inventors surprisingly found that different blending process can result in different blend uniformity issues although the composition comprises same active ingredient and same pharmaceutically acceptable excipients. Present inventors compare results which were found using geometric dilution and dry blend process to manufacture same solid dosage form.

Prior to filing, the blend uniformity of powder is analyzed. After mixing all of the ingredients in the cubic container blender, ten samples of the mixture are taken from different locations in the blender and a potency assay is determined by HPLC.

### Blend Uniformity results of Fingolimod HCl (Dry blend process)

**Table 3**

| Sample No | % |
|---|---|
| 1 | 107.20 |
| 2 | 92.50 |
| 3 | 84.30 |
| 4 | 112.90 |
| 5 | 88.90 |
| 6 | 71.10 |
| 7 | 117.60 |
| 8 | 105.40 |
| 9 | 97.30 |
| 10 | 107.20 |
| **Average** | **98.40** |
| **RSD%** | **14.50** |

The results of the potency assay are as follows: the average of ten samples is 98.4, the range is between 71.1 to 112.9, and a relative standard deviation (RSD) is 14.5.

As can be seen in the table above; due to unacceptable results of RSD%, an alternative improved method is considered necessary. Hence,the inventors of present invention have selected geometric dilution method in order to obtain suitable RSD% results.

According to the present invention, stable pharmaceutical compositions of fingolimod having good blend uniformity can be prepared by using the geometric dilution process.

### Blend Uniformity results of Fingolimod HCl (geometric dilution process)

**Table 4**

| Sample No | % |
|---|---|
| 1 | 103.50 |
| 2 | 103.30 |
| 3 | 105.50 |
| 4 | 100.90 |
| 5 | 101.00 |
| 6 | 97.00 |
| 7 | 103.30 |
| 8 | 105.40 |
| 9 | 97.00 |
| 10 | 103.40 |
| **Average** | **102.00** |
| **RSD%** | **2.99** |

The results of the potency assay are as follows: the average of ten samples is 102.0, the range is between 97.0 to 105.5, and a relative standard deviation (RSD) is 2.99.

The obtained results shown in Table 4 clearly indicate that the blend uniformity is ensured. The RSD% is below the limit.

In the case of low-dose drug products, the blend and content uniformity of the dosage form can be negatively affected if the particle size of the active pharmaceutical ingredient and particle size of the excipients are very different from each other. Even if the blend remains chemically homogeneous, variations in particle size can also affect flowability.

To optimize formulation, the present inventors have chosen that the particle size of fingolimod and particle size of calcium carboxymethylcellulose are approximately the same in order.

In the pharmaceutical industry, when powdered components are filling into the capsule, the filling speed of capsules is one of the critical factors for ensuring the content uniformity.

In another aspect of the present invention, the content uniformity results of the pharmaceutical compositions have been studied with a three different filling speed (such as 12.000 caps/h, 14.000 caps/h, 16.000 caps/h) are given in Table 5.

**Table 5**

| | **Content uniformity of the capsule with a filing of (12.000 caps/h)** | **Content uniformity of the capsule with a filing of (14.000 caps/h)** | **Content uniformity of the capsule with a filing of (10.000 caps/h)** |
|---|---|---|---|
| **1** | 105.27 | 103.47 | 102.42 |
| **2** | 102.24 | 103.34 | 99.72 |
| **3** | 102.75 | 105.46 | 100.09 |
| **4** | 105.46 | 100.89 | 102.40 |
| **5** | 109.43 | 100.95 | 97.67 |
| **6** | 102.82 | 96.97 | 91.21 |
| **7** | 102.83 | 103.26 | 97.36 |
| **8** | 102.55 | 105.36 | 97.76 |
| **9** | 103.00 | 96.98 | 91.76 |
| **10** | 109.59 | 103.42 | 97.45 |
| **Average** | **104.59** | **102.01** | **97.79** |
| **SD** | **2.82** | **3.05** | **3.82** |
| **RSD** | **2.70** | **2.99** | **3.91** |
| **AV** | **10** | **8** | **10** |

Thanks to good results of blend uniformity, the suitability particle size of fingolimod and calcium carboxymethylcellulose and the content uniformity of formulation are also good in different filling speed.

The obtained results as shown in Table 5 clearly indicate that the content uniformity is ensured. The content uniformity of capsule was high despite the small amount of active ingredient in each capsule. The RSD% is below the limit in three different filling speeds. As such, these results would pass the acceptance criteria set by the first stage of the EP (European Pharmacopeia) content uniformity test.

The formulation comprises an effective amount of fingolimod or pharmaceutically acceptable salts, esters, hydrates and solvates thereof. In the following description, any reference to the term "fingolimod" is intended to include the pharmaceutically acceptable salts, esters, hydrates and solvates thereof, and especially fingolimod hydrochloride.

"Pharmaceutically acceptable excipient(s)" are components added to active agent pharmaceutical formulation other than the active ingredient fingolimod. Excipients may be added to facilitate manufacture, enhance stability, enhance product characteristics, enhance bioavailability, enhance patient acceptability, etc.

Pharmaceutically acceptable excipient(s) includes, but not limited to, one or more of fillers, binders, disintegrants, lubricants, glidants, compression aids, colors, sweeteners, preservatives, surfactants, suspending agents, dispersing agents, film formers, flavors, printing inks, etc.

One or more these excipients can be selected and used by the artisan having regard to the particular desired properties of the solid dosage form. The amount of each type of excipient employed, e.g. glidant, binder, disintegrant, filler and lubricant may vary within ranges conventional in the art.

The term 'filler' and the term 'diluent' are herein used interchangeably. Fillers/diluents are, but not limited to, calcium phosphate, dibasic calcium phosphate, calcium carbonate, tribasic calcium sulfate, calcium carboxymethylcellulose, cellulose, dextrin derivatives, dextrin, dextrose, fructose, lactitol, lactose,methylcellulose polymers, hydroxyethylcellulose,hydroxypropylcellulose, hydroxypropyl methylcellulose, carboxymethylene ,sodium carboxymethylcellulose, carboxymethylhydroxyethylcellulose and other cellulose derivatives, starches or modified starches, magnesium carbonate, magnesium oxide, maltitol, maltodextrins, maltose, sorbitol, starch, sucrose, sugar, and xylitol, erythritol. Preferably, calcium carboxymethylcellulose is used.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pregelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Disintegrants are, but not limited to, sodium starch glycolate, croscarmellose sodium, crospovidone(cross linked polyvinylpyrolidone), alginic acid, chitosan, methyl cellulose, microcrystalline cellulose, powdered cellulose,lower alkylsubstituted hydroxypropyl cellulose, polacrilin potassium, starch, pregelatinized starch, sodium alginate, mixtures thereof or whatsoever.

Lubricants are, but not limited to, calcium stearate, glyceryl monostearate, glyceryl behenate, glyceryl palmitostearate, hydrogenated vegetable oil, light mineral oil, magnesium stearate, magnesium lauryl sulfate, mineral oil, polyethylene glycol, poloxamer, sodium benzoate, sodium lauryl sulfate, sodium stearate, sodium benzoate ,sodium stearyl fumarate, stearic acid, talc, zinc stearate, mixtures thereof and the like. Preferably, sodium stearyl fumarate is used because its hydrophobicity is weak and its delayed effect is low, and its influence on the fingolimod is low.

Glidants are, but not limited to, silicon dioxide, colloidal silicon dioxide, calcium silicate, magnesium silicate, magnesium trisilicate, talc, starch, tribasic calcium phosphate mixtures thereof and the like.

Suitable pharmaceutical compositions include, but are not limited to, capsules, tablets, granules, powders and unit dose pockets. Preferably oral pharmaceutical formulation is a capsule.

For the manufacture of solid dosage forms, for example, the active ingredients may be mixed with the excipients and/or additives and granulated in a wet or dry process. Wet or dry granulation, direct compression, melt granulation, melt congealing, extrusion process and such processes can be applied. Granules or powder can be filled directly into capsules or compressed into tablet cores.

Granulation can be achieved via spray granulation technique in which spraying and drying are performed simultaneously or using high or low shear granulation technique in which an additional drying step after granulation is necessary to remove solvent. Alternatively, one-pot processor where both high or low shear granulation and drying are performed can be used. Granulation solvent can be selected from pharmaceutically acceptable solvents or mixtures thereof. Water is the preferred solvent, and mixtures of water and other organic solvents can also be used. Granulation solvent can further contain additional functional excipients or cosolvents such as but not limited to binder(s), solubility enhancer(s), emulsifier(s), edible vegetable oil(s), absorption enhancer(s) or mixtures thereof.

Drying temperature is limited as such that the product temperature does not exceed 30°C during the complete manufacturing process. Since the product temperature did not exceed 30°C during drying,the inventors of present invention have not encountered the maillard reaction problem,one of the most common undesirable situation in the prior art.

In wet granulation, granules or powder can be filled directly into capsules or compressed into tablet cores.

In this invention the term "blend uniformity" as used herein, refers to the homogeneity of blend before converting into finished dosage form e.g. pharmaceutical compositions such as capsules. The ingredients prior to filling in capsule were evaluated in a blend uniformity study. After mixing all of the ingredients in the any type of blender, especially cubic container blender, ten samples of the mixture were removed from different locations in the blender and a potency assay was determined by HPLC.

In this invention the term "content uniformity" or "uniformity of dosage form" as used herein, refers to the homogeneity of the drug content among individual units of finished dosage form, e.g. pharmaceutical compositions such as capsules.

Although the EP (European Pharmacopeia) is not a regulatory body, their recommendations for testing protocols are readily adopted by pharmaceutical companies. In the case of content uniformity EP: 1. The actual % API in 10 capsules is tested and the acceptance value (AV) is calculated. The requirements for dosage uniformity are met if the acceptance value (AV) of the first 10 dosage units is less than or equal to L1. If the acceptance value is greater than L1, next 20 dosage units must be tested and the acceptance value must be calculated. The requirements are met if the final acceptance value of 30 dosage units is less than or equal to L1 and no individual content of the dosage unit is less than (1-L2^{∗}0.01)M or more than (1+L2^{∗}0.01)M in calculation of acceptance value under content uniformity or under mass variation. (unless otherwise specified, L1 =15.0,L2=25.0). 2. The Relative Standard Deviation (RSD) is no greater than 6.0%.

In this invention the term "relative standard deviation" or "RSD", as used herein, refers to a measurement of how precise each measurement of content uniformity is, i.e., how much each individual unit deviates from the group with regards to the drug content.

In this invention the term "standard deviation" or "SD" as used herein, refers to a statistical measurement of the precision for a series of repetitive measurements.

In this invention the term "acceptance value" or "AV" as used herein, refers to a pharmacopeia test, was applied to study the uniformity of drug distribution is a test recommended by the European Pharmacopeia.

In this invention the relative humidity is abbreviated as "RH" or "Rh".

Total % quantity of active agent(s) may vary between 0.5 - 10% of total weight of pharmaceutical dosage form.

Preferably, total % quantity of active agent(s) varies between 0.5-2.5 % of total weight of pharmaceutical dosage form.

Total % quantity of filler(s) may vary between 1 - 99% of total weight of pharmaceutical dosage form.

Preferably, total % quantity of filler(s) varies between 90- 98% of total weight of pharmaceutical dosage form.

Total % quantity of lubricant(s) may vary between 0.1 - 4% of total weight of pharmaceutical dosage form.

Preferably, total % quantity of lubricant(s) varies between 0.5- 3% of total weight of pharmaceutical dosage form.

In a further aspect, the pharmaceutical compositions are useful for treatment and prevention of autoimmune disease or of inflammatory conditions, for instance multiple sclerosis, arthritis, inflammatory bowel disease, hepatitis, etc.

### Example(s)

The following examples are given for the purpose of illustration of the present invention and should not limit the scope of the invention.

### Example 1 (Unit Formula: Capsule)

In the present example, calcium carboxymethylcellulose is used as filler.

| **Raw Material** | **mg/caps** |
|---|---|
| Fingolimod HCl | 0.56 |
| Calcium carboxymethycellulose | 48.94 |
| Sodium stearyl fumarate | 0.50 |
| Fill weight | 50.00 |
| Size 3 Hard Gelatin Capsule | 48.00 |
| Total | 98.00 |

### Example 2 (Manufacturing Process of Example 1)

(1)Dispensing raw materials separately.
(2)Sifting fingolimod HCl and calcium carboxymethycellulose through - a suitable sieve and loading into the container blender.
(3)Blending the sifted mixture for 15 minutes at 20 rpm.
(4)Sifting the sodium stearyl fumarate through a suitable sieve and add into the container blender. Blending it for 5 minutes at 20 rpm.
(5)Filling the final mixture in size 3 hard gelatin capsules with 50.0 mg ± 5.8 % (47.1 mg - 52.9 mg) average fill weight and certain capsule filling parameters.

### Example 3 (Unit Formula: Capsule)

In the present example, calcium carboxymethylcellulose is used as filler.

| Raw Material | **mg/caps** |
|---|---|
| Fingolimod HCl | 0.56 |
| Calcium carboxymethycellulose | 48.94 |
| Sodium stearyl fumarate | 0.50 |
| Fill weight | 50.00 |
| Size 3 Hard Gelatin Capsule | 48.00 |
| Total | 98.00 |

### Example 4 (Manufacturing Process of Example 3)

(1)Dispensing raw materials separately.
(2)Blending fingolimod HCl and one third (1/3) of calcium carboxymethycellulose in an appropriate container for 5 minutes.
(3)Sifting the obtained mixture with remaining calcium carboxymethycellulose (2/3) through a suitable sieve and loading into the container blender.
(4)Blending the sifted mixture for 15 minutes at 20 rpm.
(5)Sifting the sodium stearyl fumarate through a suitable sieve and add into the container blender. Blend it for 5 minutes at 20 rpm.
(6)Filling the final mixture in size 3 hard gelatin capsules with 50.0 mg ± 5.8 % (47.1 mg - 52.9 mg) average fill weight and certain capsule filling parameters.

### Example 5 (Unit Formula: Capsule)

In the present example , microcrystalline cellulose is used as filler instead of calcium carboxymethylcellulose.

| **Raw Material** | **mg/caps** |
|---|---|
| Fingolimod HCl | 0.56 |
| Microcrystalline cellulose | 48.94 |
| Sodium stearyl fumarate | 0.50 |
| Fill weight | 50.00 |
| Size 3 Hard Gelatin Capsule | 48.00 |
| Total | 98.00 |

### Example 6 (Manufacturing Process of Example 5)

(1)Dispensing raw materials separately.
(2)Blending fingolimod HCl and one third (1/3) of microcrystalline cellulose in an appropriate container for 5 minutes.
(3)Sifting the obtained mixture with remaining microcrystalline cellulose (2/3) through a suitable sieve and load into the container blender.
(4)Blend the sifted mixture for 15 minutes at 20 rpm.
(5)Sift the sodium stearyl fumarate through a suitable sieve and add into the container blender. Blend it for 5 minutes at 20 rpm.
(6)Fill the final mixture in size 3 hard gelatin capsules with 50.0 mg ± 5.8 % (47.1 mg - 52.9 mg) average fill weight and certain capsule filling parameters.

### Example 7 (Unit Formula: Capsule)

In the present example , calcium carboxymethylcellulose is used as filler.

| **Raw Material** | **mg/caps** |
|---|---|
| Fingolimod Base | 0.50 |
| Calcium carboxymethycellulose | 49.00 |
| Sodium stearyl fumarate | 0.50 |
| Fill weight | 50.00 |
| Size 3 Hard Gelatin Capsule | 48.00 |
| Total | 98.00 |

### Example 8 (Manufacturing Process of Example 7)

(1)Dispensing raw materials separately.
(2)Blending fingolimod Base and one third (1/3) of calcium carboxymethycellulose in an appropriate container for 5 minutes.
(3)Sifting the obtained mixture with remaining calcium carboxymethycellulose (2/3) through a suitable sieve and loading into the container blender.
(4)Blending the sifted mixture for 15 minutes at 20 rpm.
(5)Sifting the sodium stearyl fumarate through a suitable sieve and add into the container blender. Blend it for 5 minutes at 20 rpm.
(6)Filling the final mixture in size 3 hard gelatin capsules with 50.0 mg ± 5.8 % (47.1 mg - 52.9 mg) average fill weight and certain capsule filling parameters.

### Example 9 (Unit Formula: Capsule)

In the present example, calcium carboxymethylcellulose is used as filler.

| **Raw Material** | **mg/caps** |
|---|---|
| Fingolimod Mesylate | 0.66 |
| Calcium carboxymethycellulose | 48.84 |
| Sodium stearyl fumarate | 0.50 |
| Fill weight | 50.00 |
| Size 3 Hard Gelatin Capsule | 48.00 |
| Total | 98.00 |

### Example 10 (Manufacturing Process of Example 9)

(1)Dispensing raw materials separately.
(2)Blending fingolimod mesylate and one third (1/3) of calcium carboxymethycellulose in an appropriate container for 5 minutes.
(3)Sifting the obtained mixture with remaining calcium carboxymethycellulose (2/3) through a suitable sieve and loading into the container blender.
(4)Blending the sifted mixture for 15 minutes at 20 rpm.
(5)Sifting the sodium stearyl fumarate through a suitable sieve and add into the container blender. Blend it for 5 minutes at 20 rpm.
(6)Filling the final mixture in size 3 hard gelatin capsules with 50.0 mg ± 5.8 % (47.1 mg - 52.9 mg) average fill weight and certain capsule filling parameters.

### Example 11 (Unit Formula: Capsule)

In the present invention , calcium carboxymethylcellulose is used as filler.

| **Raw Material** | **mg/caps** |
|---|---|
| Fingolimod Besylate | 0.76 |
| Calcium carboxymethycellulose | 48.74 |
| Sodium stearyl fumarate | 0.50 |
| Fill weight | 50.00 |
| Size 3 Hard Gelatin Capsule | 48.00 |
| Total | 98.00 |

### Example 12 (Manufacturing Process of Example 11)

(1)Dispensing raw materials separately.
(2)Blending fingolimod besylate and one third (1/3) of calcium carboxymethycellulose in an appropriate container for 5 minutes.
(3)Sifting the obtained mixture with remaining calcium carboxymethycellulose (2/3) through a suitable sieve and loading into the container blender.
(4)Blending the sifted mixture for 15 minutes at 20 rpm.
(5)Sifting the sodium stearyl fumarate through a suitable sieve and add into the container blender. Blend it for 5 minutes at 20 rpm.
(6)Filling the final mixture in size 3 hard gelatin capsules with 50.0 mg ± 5.8 % (47.1 mg - 52.9 mg) average fill weight and certain capsule filling parameters.

## Claims

1. A stable pharmaceutical composition suitable for oral administration, comprising;
(a) Fingolimod or pharmaceutically acceptable salts, esters, hydrates and solvates thereof; and
(b)Calcium carboxymethylcellulose.

2. A stable pharmaceutical composition according to claim 1, wherein fingolimod is present as fingolimod hydrochloride.

3. A stable pharmaceutical composition according to any of the preceding claims, further comprising a lubricant.

4. A stable pharmaceutical composition according to claim 3, wherein the lubricant comprises sodium stearyl fumarate.

5. A stable pharmaceutical composition according to any of the preceding claims, comprising 0.1 to 20% by weight of fingolimod.

6. A stable pharmaceutical composition according to any of the preceding claims, comprising 0.5 to 5% by weight of fingolimod.

7. A stable pharmaceutical composition according to any of the preceding claims, comprising 1 to 99% by weight of calcium carboxymethylcellulose.

8. A stable pharmaceutical composition according to any of the preceding claims, comprising 90 to 98% by weight of calcium carboxymethylcellulose.

9. A stable pharmaceutical composition according to any of the preceding claims, is in the form of a tablet or capsule.

10. A method for preparing the composition according to any one of claims 4-9, comprising the steps of:
(1) dispensing the raw materials separately,
(2) blending fingolimod HCl and one third (1/3) of calcium carboxymethylcellulose in a suitable container for 5 minutes,
(3) sifting the obtained mixture with remaining calcium carboxymethylcellulose (2/3) through a suitable sieve and loading into the container blender,
(4) blending the sifted mixture for 15 minutes at 20 rpm,
(5) sifting the sodium stearyl fumarate through a suitable sieve and adding into the container blender; blending it for 5 minutes at 20 rpm,
(6) filling the final mixture in size 3 hard gelatin capsules with 50.0 mg ± 5.8% (47.1 mg - 52.9 mg) average fill weight and certain capsule filling parameters.

11. A method for preparing the composition according to any one of claims 4-9, comprising the steps of:
(1) dispensing the raw materials separately,
(2) sifting fingolimod HCl and calcium carboxymethylcellulose through a suitable sieve and load into the container blender,
(3) blending the sifted mixture for 15 minutes at 20 rpm,
(4) sifting sodium stearyl fumarate through a suitable sieve and adding into the container blender; blending it for 5 minutes at 20 rpm,
(5) filling the final mixture in size 3 hard gelatin capsules with 50.0 mg ± 5.8 % (47.1 mg - 52.9 mg) average fill weight and certain capsule filling parameters.

12. Use of the pharmaceutical composition of claims 1-9 in the preparation of a medicament for the prevention or treatment of autoimmune diseases.

13. Use of the pharmaceutical composition of claims 1-9 in the preparation of a medicament for the treatment of multiple sclerosis.

## Patentansprüche

1. Eine stabile pharmazeutische Zusammensetzung, die zur oralen Verabreichung geeignet ist, umfassend;
(a) Fingolimod oder pharmazeutisch verträgliche Salze, Ester, Hydrate und Solvate davon; und
(b) Calciumcarboxymethylcellulose.

2. Stabile pharmazeutische Zusammensetzung nach Anspruch 1, wobei Fingolimod als Fingolimod Hydrochlorid vorliegt.

3. Stabile pharmazeutische Zusammensetzung nach einem der vorhergehenden Ansprüche, weiter umfassend ein Schmiermittel.

4. Stabile pharmazeutische Zusammensetzung nach Anspruch 3, wobei das Schmiermittel Natriumstearylfumarat umfasst.

5. Stabile pharmazeutische Zusammensetzung nach einem der vorhergehenden Ansprüche, umfassend 0.1 bis 20 Gew.-% von Fingolimod.

6. Stabile pharmazeutische Zusammensetzung nach einem der vorhergehenden Ansprüche, umfassend 0.5 bis 5 Gew.-% von Fingolimod.

7. Stabile pharmazeutische Zusammensetzung nach einem der vorhergehenden Ansprüche, umfassend 1 bis 99 Gew.-% von Calciumcarboxymethylcellulose.

8. Stabile pharmazeutische Zusammensetzung nach einem der vorhergehenden Ansprüche, umfassend 90 bis 98 Gew.-% von Calciumcarboxymethylcellulose.

9. Stabile pharmazeutische Zusammensetzung nach einem der vorhergehenden Ansprüche, welche in der Form einer Tablette oder einer Kapsel vorliegt.

10. Verfahren zur Herstellung der Zusammensetzung nach einem der Ansprüche 4 bis 9, umfassend die Schritte:
(1) getrennt, die Rohstoffe Abgeben,
(2) Mischen von Fingolimod-HCl und einem Drittel (1/3) von Calciumcarboxymethylcellulose in einem geeigneten Behälter für 5 Minuten,
(3) Sieben der erhaltenen Mischung mit verbleibender Calciumcarboxymethylcellulose (2/3) durch ein geeignetes Sieb und Laden in den Behältermischer,
(4) Mischen der gesiebten Mischung für 15 Minuten bei 20 U/min,
(5) Sieben von Natriumstearylfumarat durch ein geeignetes Sieb und Hinzufügen in den Behältermischer; Mischen für 5 Minuten bei 20 U / min,
(6) Füllen der Endmischung in Hartgelatinekapseln der Größe 3 mit 50,0 mg ± 5,8% (47,1 mg - 52,9 mg) durchschnittlichem Füllgewicht und bestimmten Kapselfüllparametern.

11. Verfahren zur Herstellung der Zusammensetzung nach einem der Ansprüche 4 bis 9, umfassend die Schritte:
(1) getrennt, die Rohstoffe Abgeben,
(2) Sieben von Fingolimod HCl und Calciumcarboxymethylcellulose durch ein geeignetes Sieb und und Laden in den Behältermischer,
(3) Mischen der gesiebten Mischung für 15 Minuten bei 20 U/min,
(4) Sieben von Natriumstearylfumarat durch ein geeignetes Sieb und Hinzufügen in den Behältermischer; Mischen für 5 Minuten bei 20 U/min,
(5) Füllen der Endmischung in Hartgelatinekapseln der Größe 3 mit 50,0 mg ± 5,8% (47,1 mg - 52,9 mg) durchschnittlichem Füllgewicht und bestimmten Kapselfüllparametern.

12. Verwendung der pharmazeutische Zusammensetzung nach Ansprüche 1-9 zur Herstellung eines Arzneimittels für die Prävention oder Behandlung von Autoimmunkrankheiten.

13. Verwendung der pharmazeutische Zusammensetzung nach Ansprüche 1-9 zur Herstellung eines Arzneimittels für die Behandlung von multipler Sklerose.

## Revendications

1. Composition pharmaceutique stable appropriée à l'administration par voie orale, comprenant;
(a) Fingolimod ou ses sels, esters, hydrates et solvates pharmaceutiquement acceptables; et
(b) carboxyméthylcellulose de calcium.

2. Composition pharmaceutique stable selon la revendication 1, où fingolimod est présent sous forme de chlorhydrate de fingolimod.

3. Composition pharmaceutique stable selon l'une quelconque des revendications précédentes, comprenant en outre un lubrifiant.

4. Composition pharmaceutique stable selon la revendication 3, dans lequelle le lubrifiant comprend du stéarylfumarate de sodium.

5. Composition pharmaceutique stable selon l'une quelconque des revendications précédentes, comprenant de 0,1 à 20% en poids de fingolimod.

6. Composition pharmaceutique stable selon l'une quelconque des revendications précédentes, comprenant de 0.5 à 5% en poids de fingolimod.

7. Composition pharmaceutique stable selon l'une quelconque des revendications précédentes, comprenant de 1 à 99% en poids de carboxyméthylcellulose de calcium.

8. Composition pharmaceutique stable selon l'une quelconque des revendications précédentes, comprenant de 90 à 98% en poids de carboxyméthylcellulose de calcium.

9. Composition pharmaceutique stable selon l'une quelconque des revendications précédentes, se présente sous la forme d'un comprimé ou d'une capsule.

10. Procédé de préparation de la composition selon l'une quelconque des revendications 4 à 9, comprenant les étapes consistant à:
(1) dispenser les matières premières séparément,
(2) mélanger du fingolimod HCl et un tiers (1/3) de carboxyméthylcellulose de calcium dans un récipient approprié pendant 5 minutes,
(3) tamiser le mélange obtenu avec la carboxyméthylcellulose de calcium restante (2/3) à travers un tamis approprié et charger dans le mélangeur à récipients,
(4) mélanger le mélange tamisé pendant 15 minutes à 20 tours par minute,
(5) tamiser le fumarate de stéaryle de sodium à travers un tamis approprié et ajouter dans le mélangeur à récipients; mélanger pendant 5 minutes à 20 tours par minute,
(6) remplisser du mélange final dans des capsules de gélatine dure de taille 3 avec 50,0 mg ± 5,8% (47,1 mg - 52,9 mg) de poids de remplissage moyen et certains paramètres de remplissage de la capsule.

11. Procédé de préparation de la composition selon l'une quelconque des revendications 4 à 9, comprenant les étapes consistant à:
(1) dispenser les matières premières séparément,
(2) tamiser le fingolimod HCl et la carboxyméthylcellulose de calcium à travers un tamis approprié et les charger dans le mélangeur de récipients,
(3) mélanger le mélange tamisé pendant 15 minutes à 20 tours par minute,
(4) tamiser le fumarate de stéaryle de sodium à travers un tamis approprié et ajouter dans le mélangeur à récipients; mélanger pendant 5 minutes à 20 tours par minute,
(5) remplisser du mélange final dans des capsules de gélatine dure de taille 3 avec 50,0 mg ± 5,8% (47,1 mg - 52,9 mg) de poids de remplissage moyen et certains paramètres de remplissage de la capsule.

12. Utilisation de la composition pharmaceutique des revendications 1 à 9 dans la préparation d'un médicament pour la prévention ou le traitement de maladies auto-immunes.

13. Utilisation de la composition pharmaceutique des revendications 1 à 9 dans la préparation d'un médicament pour le traitement de la sclérose en plaques.
